# EUROPEAN PATENT APPLICATION

(11) **EP 4 830 630 A1**
(43) Date of publication of application: **28.10.2026**
(21) Application number: 24906985.7
(22) Date of filing: 12.11.2024
(51) Int. Cl.: A61B 5/256, A61B 5/291

(54) **BRAIN WAVE MEASUREMENT DEVICE AND BRAIN WAVE MEASUREMENT METHOD**

(30) Priority: 19.12.2023 JP 2023213993
(71) Applicant: Sumitomo Bakelite Co.Ltd., Shinagawa-ku Tokyo 140-0002 (JP)
(72) Inventor: KITAZOE, Katsuma, Tokyo 140-0002 (JP); ISHIKAWA, Eiji, Tokyo 140-0002 (JP)
(74) Representative: Ter Meer und Partner mbB
(86) International application number: PCT/JP2024/040115
(87) International publication number: WO 2025/134597

(57) **Abstract**

A brain wave measurement device includes an electrode unit configured to contact a measurement site on a head of a subject and acquire a brain wave signal, a support member (20) configured to support the electrode unit, and an attachment part (70) (assisting member) configured to assist placement of the support member (20) on a head, in which the support member (20) has a non-stretchable film member and a mounting portion (25) to which the electrode unit is electrically connected and fixed, the film member has a non-stretchable base material and a circuit pattern provided on the base material, and the circuit pattern is connected to the mounting portion (25).

## Description

### TECHNICAL FIELD

The present invention relates to a brain wave measurement device and a brain wave measurement method.

### BACKGROUND ART

Various developments have been made on a brain wave measurement device which measures brain waves. As a kind of this technology, for example, a brain wave measurement device has been known, that includes a support which is made of a shape memory material and which is a head band mounted on a head of a user, and a vital sensor which is attached to the support and acquires a biological signal of the user (see, for example,

Patent Document 1). According to the technology of Patent Document 1, in a case of measuring a biological signal, the support can be easily restored to a shape that matches the body shape of the user in which the shape of the support is stored in advance.

### RELATED DOCUMENT

### PATENT DOCUMENT

Patent Document 1: Japanese Patent No. 5900167

### SUMMARY OF THE INVENTION

### TECHNICAL PROBLEM

However, in the brain wave measurement, there has been a concern that, in order to bring a plurality of electrodes into contact with the head (that is, the scalp), a large number of signal lines connected to the electrodes are required, so that the device as a whole becomes complicated.

The technology disclosed in Patent Document 1 does not take a wiring structure into consideration, and there has been a need for a new technology.

The present invention has been made in view of such circumstances, and an object of the present invention is to provide a technology for simplifying a wiring structure connected to electrodes of a brain wave measurement device.

### SOLUTION TO PROBLEM

According to the present invention, the following technologies are provided.
(1) A brain wave measurement device including:
   an electrode unit configured to contact a measurement site on a head of a subject and acquire a brain wave signal;
   a support member configured to support the electrode unit; and
   an assisting member configured to assist placement of the support member on the head,
   in which the support member has a non-stretchable film member and a mounting portion to which the electrode unit is electrically connected and fixed,
   the film member has a non-stretchable base material and a circuit pattern provided on the base material, and
   the circuit pattern is connected to the mounting portion.
(2) The brain wave measurement device according to (1), further including:
   an electromagnetic wave shielding member configured to cover the circuit pattern.
(3) The brain wave measurement device according to (1) or (2),
   in which the film member has an elongated shape, and
   a plurality of electrode units are arranged at predetermined intervals in a longitudinal direction of the film member.
(4) The brain wave measurement device according to any one of (1) to (3),
   in which the electrode unit has a base portion, a plurality of protruding portions provided on the base portion, and an electrode portion provided on the protruding portion and in contact with the head.
(5) The brain wave measurement device according to (4),
   in which the protruding portions are elastic members.
(6) The brain wave measurement device according to any one of (1) to (5),
   in which the assisting member is provided at an end portion of the support member, is configured to be attached to an ear or a jaw, and assists placement of the support member along a shape of the head.
(7) The brain wave measurement device according to any one of (1) to (6),
   in which the assisting member includes a member having spring properties.
(8) The brain wave measurement device 10 according to any one of (1) to (7),
   in which the assisting member includes a member for adjusting a length.
(9) The brain wave measurement device 10 according to any one of (1) to (8),
   in which the support member is bent at a vertex and linearly connects the vertices, each attachment position of the electrode unit serving as the vertex.
(10) A brain wave measurement method including:
   mounting the brain wave measurement device according to any one of (1) to (9) on a head of a subject and measuring brain waves.

### ADVANTAGEOUS EFFECTS OF INVENTION

According to the present invention, it is possible to provide a technology for simplifying a wiring structure connected to electrodes of a brain wave measurement device.

### BRIEF DESCRIPTION OF THE DRAWINGS

Fig. 1 is a schematic view showing a state in which a brain wave measurement device according to an embodiment is mounted on a head.
Fig. 2 is a view showing a support member that supports an electrode unit and an attachment part (assisting member) according to the embodiment.
Fig. 3 is a view showing a circuit pattern of the support member according to the embodiment.
Fig. 4 is a cross-sectional view of the electrode unit in a state of being attached to the support member according to the embodiment.
Fig. 5 is a cross-sectional view of the electrode unit in a state of being attached to the support member according to a modification example of the embodiment.
Fig. 6 is a cross-sectional view of the support member according to the embodiment.
Fig. 7 is a view schematically showing a tension display portion according to the embodiment.
Fig. 8 is a view for explaining, based on a model, a force acting on the head when the brain wave measurement device according to the embodiment is mounted.
Fig. 9 is a view for explaining a capstan principle (equation) used for modeling according to the embodiment.
Fig. 10 is a graph showing theoretical values (calculated values) and measured values of an electrode pressing force F according to Examples.

### DESCRIPTION OF EMBODIMENTS

Hereinafter, embodiments of the present invention will be described with reference to the drawings. In the present embodiment, a brain wave measurement device which includes an electrode unit and is mounted on a head of a subject to acquire brain waves, and a brain wave measurement method using the brain wave measurement device will be described.

### <Schematic Structure of Brain Wave Measurement Device>

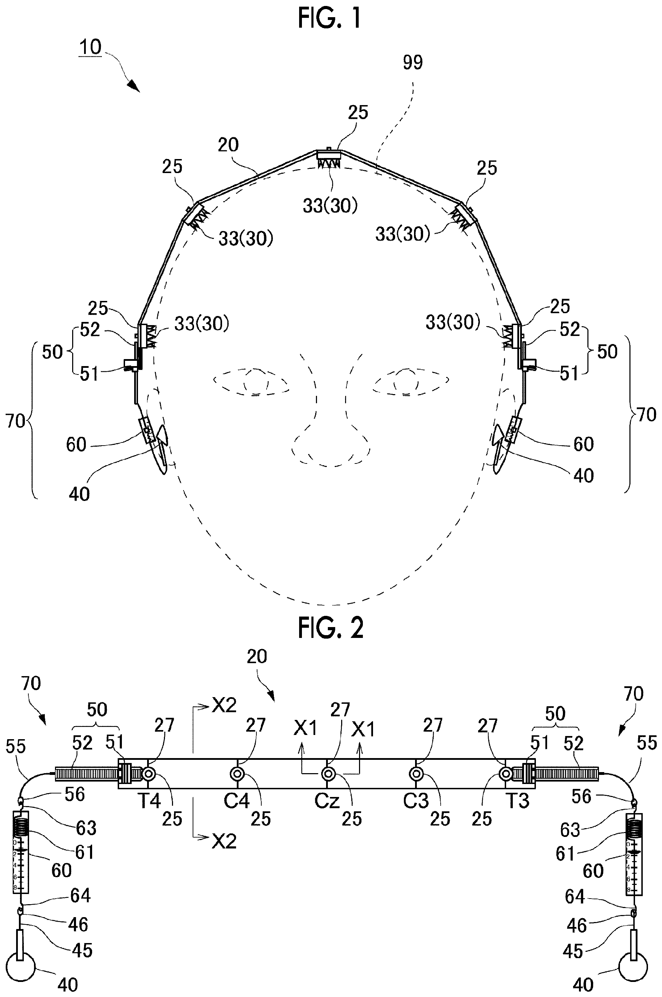

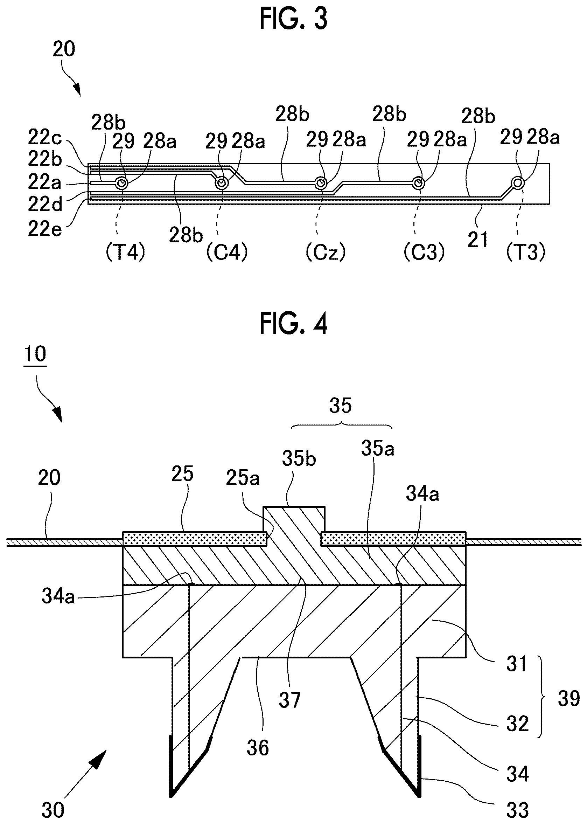

Fig. 1 is a schematic view of a state in which a brain wave measurement device 10 is mounted on a head 99 of a person, as viewed from the front. Fig. 2 is a view showing a support member 20 that supports an electrode unit 30, and an attachment part 70 (assisting member). In the present embodiment, the brain wave measurement device 10 that measures brain waves at electrode positions Cz, C3, C4, T3, and T4 (international 10-20 method) is described. Fig. 3 is a view showing a circuit pattern 22 of the support member 20. Fig. 4 is a cross-sectional view of the electrode unit 30 in a state of being attached to the support member 20.

The brain wave measurement device 10 is mounted on the head 99 of a person, detects brain waves as potential fluctuations from a living body, and outputs the detected brain waves to a brain wave display device (not shown). The brain wave display device acquires the brain waves detected by the brain wave measurement device 10, displays them on a monitor, stores data of them, and performs a known brain wave analysis process (measurement process).

The brain wave measurement device 10 has a plurality of electrode units 30 configured to contact a measurement site (that is, the head 99) of a subject and acquire brain waves which are biological signals, a support member 20 having a non-stretchable film member (also referred to as an elongated sheet-like member or a ribbon member) configured to attach and support the electrode units 30, and an attachment part 70 used for fixing the support member 20 to the head 99. In the present embodiment, the attachment part 70 is mounted on an ear of the subject, thereby functioning as an assisting member for fixing the brain wave measurement device 10 to the head 99. The attachment part 70 has a tension display portion 60. The tension display portion 60 indicates a tension acting on the support member 20 in a recognizable manner. Non-stretchability of the film member is defined by a Poisson's ratio, which will be described later, being in a predetermined range.

The support member 20 (film member) has a circuit pattern 22 and a mounting portion 25. The mounting portion 25 is configured such that the electrode unit 30 is electrically connected and fixed thereto. The circuit pattern 22 is connected to the electrode unit 30 and an external brain wave display device or the like through the mounting portion 25. By adopting such a structure, a wiring structure of the brain wave measurement device 10 is organized in an orderly manner. In order to achieve such a structure, a flexible circuit (also referred to as a flexible substrate) is used as the support member 20.

The support member 20 is configured to be bendable to some extent at least in a thickness direction. In addition, a member (so-called hard member such as a helmet) which is provided in advance with a shape by surrounding the support member 20 is not provided. In a state of not being mounted on the head 99, the support member 20 and the like can be in a flat state without particularly applying an external force, and in a case of being mounted on the head 99, it has flexibility such that a shape thereof changes to follow the shape of the head 99 mainly by gravity. In the present embodiment, the support member 20 is bent at the position of the electrode unit 30 (mounting portion 25) such that a polygon (or a polygonal line) is formed in which the electrode unit 30 (mounting portion 25) serves as a vertex and a side connecting the vertices is the support member 20. Since both end portions of the electrode unit 30 may be in a state of being lifted from the head 99 due to influence of head hair or the like, the attachment part 70 is used for supporting the follow-up to the shape of the head 99.

The support member 20 has a female snap button 25a formed of a highly conductive metal as the mounting portion 25. A male snap button 35 of the electrode unit 30 is fitted into the female snap button 25a, thereby fixing the electrode unit 30 to the support member 20. The female snap button 25a has a sufficient strength such that the electrode unit 30 is stably fixed. The strength is appropriately adjusted according to the force acting on the electrode unit 30. In addition, since the female snap button 25a is formed of a highly conductive metal, when the male snap button 35 of the electrode unit 30 is fitted into the female snap button 25a, the electrode unit 30 is electrically connected to the circuit pattern 22 through the female snap button 25a.

In the support member 20, a position of the female snap button 25a in the left-right direction coincides with a bent portion 27 where the support member 20 is bent. By providing the bent portion 27, an extending direction of the support member 20 between the electrode units 30 can be made linear. By providing the female snap button 25a, the bent portion 27 may not be completely bent and may be in a deformed state including a curve or the like with a certain width; but even in that case, it is sufficient that the deformation is to an extent that a capstan principle (capstan equation) to be described later can be applied. This is appropriately adjusted by a thickness of the female snap button 25a or a base material 21, and the like. In addition, when the support member 20 is sufficiently thin such that, even without the bent portion 27, deformation to an extent sufficient for application of the capstan principle (capstan equation) can be achieved, the bent portion 27 may be omitted.

The attachment part 70 includes an ear attachment portion 40, an adjustment part 50, and the tension display portion 60, and these are connected by a member (here, cords 45 and 55) that does not extend.

When the brain wave measurement device 10 is mounted on the head 99, the positions of the electrode units 30 are substantially the vertices of the polygon, and a shape is formed in which the support member 20 spans between the electrode units 30. That is, the support member 20 is bent at a portion (bent portion 27) that coincides with a portion to which the electrode unit 30 is attached. The bent portion 27 corresponds to a position corresponding to a vertex of the polygon. A force acting on the head 99 (electrode pressing force F to the head) is calculated by the capstan principle (capstan equation) modeled using the polygon. This will be described later.

Hereinafter, each component will be specifically described.

### <Electrode Unit>

The electrode unit 30 is attachably and detachably provided only at a portion required for the brain wave measurement. Attachment positions of the electrode units 30 correspond to, for example, positions T3, C3, Cz, C4, and T4 in the international 10-20 electrode placement method, and are arranged symmetrically in a front view as shown in Fig. 1.

The electrode unit 30 is configured as a so-called button electrode as shown in Fig. 4, for example. Specifically, the electrode unit 30 has a columnar base portion 31, a protrusion portion 32 integrally provided at one end (here, a base portion lower surface 36) of the base portion 31, a conductive contact portion 33 (electrode), a signal line portion 34, and a male snap button 35 provided at the other end (here, a base portion upper surface 37) of the base portion 31. Hereinafter, the base portion 31 and the protrusion portion 32 will be referred to as an electrode portion main body 39 for convenience. The male snap button 35 is detachably attached to the female snap button 25a provided at a predetermined position of the support member 20.

The electrode unit 30 may be configured with a conductive metal as a whole, or may have a configuration in which a conductive member is provided on a surface of the electrode unit based on a rubber-like elastic member. Hereinafter, the configuration based on the rubber-like elastic member will be exemplified.

The detailed structure and material for the electrode unit 30 will be described later, but the electrode unit 30 according to the present embodiment is an electrode (dry electrode) which does not use a so-called paste for a brain wave electrode in order to ensure conductivity. However, a method of forming a gel on the dry electrode tip (tip of the protrusion portion 32) and immersing the gel in a wetting liquid or the like to secure moisture may be adopted. In addition, a method of wetting the dry electrode tip with a conductive auxiliary liquid in which a small amount of an electrolyte such as salt is mixed with a low-viscosity liquid such as a lotion, instead of the paste or a grease, may be adopted. That is, the dry electrode according to the present embodiment is not limited to the completely dry electrode, but is intended not to use an auxiliary agent such as a paste in which significant stains remain.

The electrode portion main body 39 is integrally provided by a rubber-like elastic member. The specific material for the elastic member will be described later. The electrode portion main body 39 (that is, the base portion 31 and the protrusion portion 32) is not limited to the configuration of being integrally provided, and may be configured by assembling those provided separately using an adhesive or a joining structure.

### <Shape of Base Portion and Protrusion Portion>

The base portion 31 has a substantially columnar shape. A plurality of substantially conical protrusion portions 32 which protrude in a downward direction in the drawing are provided on the circular base portion lower surface 36 on one end side of the base portion 31 (the lower side in the drawing). It is sufficient that the base portion 31 have the columnar shape, and a cross section thereof may have a shape other than a circle, such as a polygonal shape. The shape of the protrusion portion 32 is not limited to the conical shape, and various shapes of a pyramid such as a triangular pyramid, a columnar shape, and the like may be adopted.

The conductive contact portion 33 is provided on at least a distal end side surface of the protrusion portion 32. The conductive contact portion 33 may be provided on the entire surface of the protrusion portion 32. The conductive contact portion 33 is formed in a thin film shape, and in a state where the conductive contact portion 33 is provided on the protrusion portion 32, the protrusion portion 32 with the conductive contact portion 33 can be regarded as having substantially the same shape as the shape of only the protrusion portion 32.

The outer diameter of the base portion 31 is, for example, 10 mm to 50 mm. A height (thickness) of the base portion 31 is, for example, 0.1 mm to 30 mm. A height of the protrusion portion 32 is, for example, 1 mm to 20 mm. The width (outer diameter of the root portion) of the protrusion portion 32 is, for example, 1 mm to 10 mm.

### <Material for Electrode Portion Main Body (Base Portion and Protrusion Portion)>

A material for the electrode portion main body 39 will be described. The electrode portion main body 39 may be made of a rubber-like elastic body as described above. Specifically, the rubber-like elastic body is a rubber or a thermoplastic elastomer (also simply referred to as "elastomer (TPE)"). Examples of the rubber include a silicone rubber. Examples of the thermoplastic elastomer include styrene-based TPE (TPS), olefin-based TPE (TPO), vinyl chloride-based TPE (TPVC), urethane-based TPE (TPU), ester-based TPE (TPEE), amide-based TPE (TPAE), and the like.

In a case where a material for the electrode portion main body 39 is silicone rubber, a rubber hardness A is, for example, 15 or more and 55 or less in a case where a type A durometer hardness of the surface of the electrode portion main body 39 is measured at 37°C in accordance with JIS K 6253 (1997) and is defined as the rubber hardness A.

Here, the above-described silicone rubber-based curable composition will be described.

The above-described silicone rubber can be formed of a cured product of the silicone rubber-based curable composition. A curing step of the silicone rubber-based curable resin composition is performed by heating (primary curing) the composition, for example, at 100°C to 250°C for 1 to 30 minutes and post-baking (secondary curing) the heated composition at 100°C to 200°C for 1 to 4 hours.

Insulating silicone rubber is silicone rubber not containing a conductive filler, and conductive silicone rubber is silicone rubber containing a conductive filler.

The silicone rubber-based curable composition according to the present embodiment may contain a vinyl group-containing organopolysiloxane (A). The vinyl group-containing organopolysiloxane (A) is a polymer contained in the silicone rubber-based curable composition according to the present embodiment as a main component.

The insulating silicone rubber-based curable composition and the conductive silicone rubber-based curable composition may contain the same kind of vinyl group-containing linear organopolysiloxane. It is sufficient that the same kind of vinyl group-containing linear organopolysiloxane includes at least a vinyl group having the same functional group and is linear, and it may have different vinyl group amounts or molecular weight distributions in the molecule, or different addition amounts thereof.

The insulating silicone rubber-based curable composition and the conductive silicone rubber-based curable composition may further contain different kinds of vinyl group-containing organopolysiloxanes.

The vinyl group-containing organopolysiloxane (A) can contain a vinyl group-containing linear organopolysiloxane (A1) having a linear structure.

The above-described vinyl group-containing linear organopolysiloxane (A1) has a linear structure and contains a vinyl group, in which the vinyl group functions as a crosslinking point during the curing.

The content of the vinyl group in the vinyl group-containing linear organopolysiloxane (A1) is not particularly limited. For example, the vinyl group-containing linear organopolysiloxane (A1) has 2 or more vinyl groups in a molecule, and the content of the vinyl groups is preferably 15 mol% or less and more preferably 0.01 to 12 mol%. In a case where the content of the vinyl group is in the above range, the amount of the vinyl group in the vinyl group-containing linear organopolysiloxane (A1) can be optimized, and a network between the respective components, which will be described later, can be reliably formed. In the present embodiment, "to" means that the range includes numerical values as an upper limit value and a lower limit value.

In the present specification, the content of the vinyl group represents mol% of a vinyl group-containing siloxane unit with respect to 100 mol% of all units forming the vinyl group-containing linear organopolysiloxane (A1). Here, it is assumed that there may be one vinyl group for each vinyl group-containing siloxane unit.

In addition, a polymerization degree of the vinyl group-containing linear organopolysiloxane (A1) is not particularly limited, and is, for example, preferably in a range of approximately 1,000 to 10,000 and more preferably in a range of approximately 2,000 to 5,000. The polymerization degree can be obtained as, for example, a number-average polymerization degree (or number-average molecular weight) in terms of polystyrene in gel permeation chromatography (GPC) in which chloroform is used as an eluent.

Furthermore, a specific gravity of the vinyl group-containing linear organopolysiloxane (A1) is not particularly limited and is preferably in a range of approximately 0.9 to 1.1.

Using the vinyl group-containing linear organopolysiloxane (A1) having the polymerization degree and the specific gravity in the ranges described above makes it possible to obtain silicone rubber having higher heat resistance, higher flame retardancy, higher chemical stability, and the like.

It is particularly preferable that the vinyl group-containing linear organopolysiloxane (A1) has a structure represented by Formula (1).

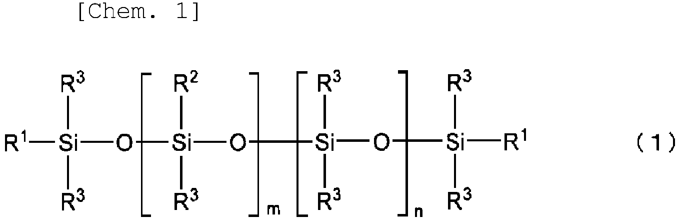

In Formula (1), R¹ represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, a butenyl group, and the like. Among these, a vinyl group is preferable. Examples of the aryl group having 1 to 10 carbon atoms include a phenyl group.

In addition, R² represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

R³ represents a substituted or unsubstituted alkyl group or aryl group having 1 to 8 carbon atoms, or a hydrocarbon group as a combination of these. Examples of the alkyl group having 1 to 8 carbon atoms include a methyl group, an ethyl group, and a propyl group. Among these, a methyl group is preferable. Examples of the aryl group having 1 to 8 carbon atoms include a phenyl group.

Furthermore, in Formula (1), examples of a substituent of R¹ and R² include a methyl group and a vinyl group, and examples of a substituent of R³ include a methyl group.

In Formula (1), a plurality of R¹'s is independent of each other, and may be the same as or different from each other. Furthermore, the same can be applied to R² and R³.

Furthermore, m and n are the number of repeating units constituting the vinyl group-containing linear organopolysiloxane (A1) represented by Formula (1), m is an integer of 0 to 2,000, and n is an integer of 1,000 to 10,000, where m is preferably 0 to 1,000 and n is preferably 2,000 to 5,000.

Examples of specific structures of the vinyl group-containing linear organopolysiloxane (A1) represented by Formula (1) include a structure represented by Formula (1-1).

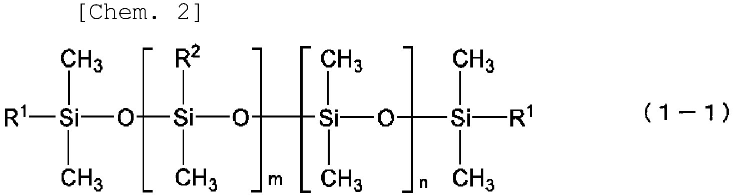

In Formula (1-1), R¹ and R² each independently represent a methyl group or a vinyl group, and at least one thereof represents a vinyl group.

Furthermore, as the vinyl group-containing linear organopolysiloxane (A1), it is preferable to contain a first vinyl group-containing linear organopolysiloxane (A1-1) having two or more vinyl groups in the molecule, in which the content of the vinyl group is 0.4 mol% or less, and a second vinyl group-containing linear organopolysiloxane (A1-2) in which the content of the vinyl group is 0.5 to 15 mol%. Combining the first vinyl group-containing linear organopolysiloxane (A1-1) having the general vinyl group content and the second vinyl group-containing linear organopolysiloxane (A1-2) having a high vinyl group content as raw rubber that is a raw material for the silicone rubber enables vinyl groups to be unevenly distributed, which makes it possible to more effectively form variations in the crosslinking density in the crosslinked network of the silicone rubber. As a result, the tear strength of the silicone rubber can be more effectively improved.

Specifically, as the vinyl group-containing linear organopolysiloxane (A1), for example, it is preferable to use the first vinyl group-containing linear organopolysiloxane (A1-1) in which, in Formula (1-1) above, two or more units of an unit in which R¹ is a vinyl group and/or an unit in which R² is a vinyl group are included in the molecule and the content of the units is 0.4 mol% or less, and the second vinyl group-containing linear organopolysiloxane (A1-2) in which the content of the unit in which R¹ is a vinyl group and/or the unit in which R² is 0.5 to 15 mol%.

In addition, in the first vinyl group-containing linear organopolysiloxane (A1-1), the content of the vinyl group is preferably 0.01 to 0.2 mol%. In addition, the second vinyl group-containing linear organopolysiloxane (A1-2) preferably has a vinyl group content of 0.8 to 12 mol%.

Furthermore, in a case where the first vinyl group-containing linear organopolysiloxane (A1-1) and the second vinyl group-containing linear organopolysiloxane (A1-2) are blended in combination, a ratio between (A1-1) and (A1-2) is not particularly limited. However, a weight ratio (A1-1):(A1-2) is preferably 50:50 to 95:5, and more preferably 80:20 to 90:10.

As each of the first vinyl group-containing linear organopolysiloxane (A1-1) and the second vinyl group-containing linear organopolysiloxane (A1-2), only one kind may be used or two or more kinds may be used in combination.

The vinyl group-containing organopolysiloxane (A) may contain a vinyl group-containing branched organopolysiloxane (A2) having a branched structure.

### <<Organohydrogen Polysiloxane (B)>>

The silicone rubber-based curable composition of the present embodiment may contain a crosslinking agent. The crosslinking agent may contain an organohydrogen polysiloxane (B).

The organohydrogen polysiloxane (B) is classified into a linear organohydrogen polysiloxane (B1) having a linear structure and a branched organohydrogen polysiloxane (B2) having a branched structure, and can include either or both of these.

The insulating silicone rubber-based curable composition and the conductive silicone rubber-based curable composition may contain the same kind of crosslinking agent. It is sufficient that the same kind of crosslinking agent has at least a common structure such as a linear structure or a branched structure, and it may have different molecular weight distributions in the molecule, different functional groups, or different addition amounts thereof.

The insulating silicone rubber-based curable composition and the conductive silicone rubber-based curable composition may further contain different types of crosslinking agents.

The linear organohydrogen polysiloxane (B1) is a polymer that has a linear structure and a structure (≡Si-H) in which hydrogen is directly bonded to Si, and undergoes a hydrosilylation reaction with vinyl groups contained in the components blended with the silicone rubber-based curable composition in addition to the vinyl groups of the vinyl group-containing organopolysiloxane (A) to crosslink the components.

The molecular weight of the linear organohydrogen polysiloxane (B1) is not particularly limited. For example, the weight-average molecular weight thereof is preferably less than or equal to 20,000 and more preferably greater than or equal to 1,000 and less than or equal to 10,000.

The weight-average molecular weight of the linear organohydrogen polysiloxane (B1) can be measured as a polystyrene-equivalent value by gel permeation chromatography (GPC) using chloroform as an eluent.

In addition, it is preferable that the linear organohydrogen polysiloxane (B1) does not have a vinyl group typically. As a result, the crosslinking reaction in the molecule of the linear organohydrogen polysiloxane (B1) can be reliably prevented from progressing.

As such a linear organohydrogen polysiloxane (B1), for example, it is preferable to use an organohydrogen polysiloxane having a structure represented by Formula (2).

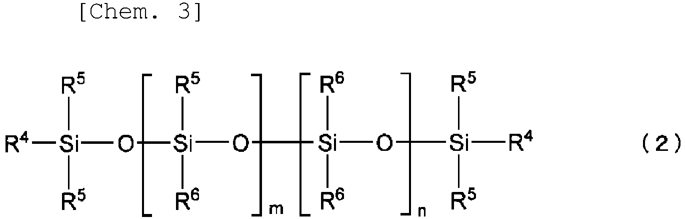

In Formula (2), R⁴ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, R⁵ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In Formula (2), a plurality of R⁴'s may be independent from each other, and may be the same or different from each other. The same can be applied to R⁵. However, at least two or more of a plurality of R⁴'s and R⁵'s represent a hydride group.

R⁶ represents a substituted or unsubstituted alkyl group or aryl group having 1 to 8 carbon atoms, or a hydrocarbon group as a combination of these. Examples of the alkyl group having 1 to 8 carbon atoms include a methyl group, an ethyl group, and a propyl group. Among these, a methyl group is preferable. Examples of the aryl group having 1 to 8 carbon atoms include a phenyl group. A plurality of R⁶'s are independent of each other, and may be the same as or different from each other.

Examples of a substituent of R⁴, R⁵, and R⁶ in Formula (2) include a methyl group and a vinyl group, and from the viewpoint of preventing the crosslinking reaction in the molecule, a methyl group is preferable.

Furthermore, m and n each independently represent the number of repeating units forming the linear organohydrogen polysiloxane (B1) represented by Formula (2), and m represents an integer of 2 to 150 and n represents an integer of 2 to 150. It is preferable that m represents an integer of 2 to 100 and n represents an integer of 2 to 100.

As the linear organohydrogen polysiloxane (B1), only one kind may be used alone, or two or more kinds may be used in combination.

Since the branched organohydrogen polysiloxane (B2) has a branched structure, it is a component that largely contributes to formation of a structure with variations in crosslinking density in the silicone rubber system by forming a region having a high crosslinking density. In addition, as in the above-described linear organohydrogen polysiloxane (B1), the branched organohydrogen polysiloxane (B2) is a polymer that has a structure in which hydrogen is directly bonded to Si (≡Si-H), and that undergoes a hydrosilylation reaction with the vinyl group in the vinyl group-containing organopolysiloxane (A) and with a vinyl group in a component blended in the silicone rubber-based curable composition to crosslink the components.

In addition, a specific gravity of the branched organohydrogen polysiloxane (B2) is in a range of 0.9 to 0.95.

It is preferable that the branched organohydrogen polysiloxane (B2) contains no vinyl group in general. As a result, the crosslinking reaction in the molecule of the branched organohydrogen polysiloxane (B2) can be reliably prevented from progressing.

In addition, it is preferable that the branched organohydrogen polysiloxane (B2) is represented by Average compositional formula (c) below.

Average compositional formula (c)

(Hₐ(R⁷)₃₋ₐSiO_{1/2})ₘ(SiO_{4/2})ₙ

(in Formula (c), R⁷ represents a monovalent organic group, a represents an integer in a range of 1 to 3, m represents the number of Hₐ(R⁷)₃₋ₐSiO_{1/2} units, and n represents the number of SiO_{4/2} units)

In Formula (c), R⁷ represents a monovalent organic group, and preferably represents a substituted or unsubstituted alkyl group or aryl group having 1 to 10 carbon atoms, or a hydrocarbon group including a combination thereof. Examples of the alkyl group having 1 to 10 carbon atoms include a methyl group, an ethyl group, and a propyl group. Among these, a methyl group is preferable. Examples of the aryl group having 1 to 10 carbon atoms include a phenyl group.

In Formula (c), a represents the number of hydride groups (hydrogen atoms directly bonded to Si) which is an integer of 1 to 3 and preferably 1.

In addition, in Formula (c), m represents the number of Hₐ(R⁷)₃₋ₐSiO_{1/2} units, and n represents the number of SiO_{4/2} units.

The branched organohydrogen polysiloxane (B2) has a branched structure. The difference between the linear organohydrogen polysiloxane (B1) and the branched organohydrogen polysiloxane (B2) is whether the structure thereof is linear or branched. Assuming that the number of Si is 1, the number of alkyl groups R bonded to Si (R/Si) is in a range of 1.8 to 2.1 in the linear organohydrogen polysiloxane (B1) and is in a range of 0.8 to 1.7 in the branched organohydrogen polysiloxane (B2).

Since the branched organohydrogen polysiloxane (B2) has a branched structure, an amount of residues is 5% or more, for example, after heating to 1,000°C at a temperature rising rate of 10 °C/min in a nitrogen atmosphere. On the other hand, since the linear organohydrogen polysiloxane (B1) is linear, an amount of residues after the heating under the above-described conditions is substantially 0.

In addition, specific examples of the branched organohydrogen polysiloxane (B2) include an organohydrogen polysiloxane having a structure represented by Formula (3).

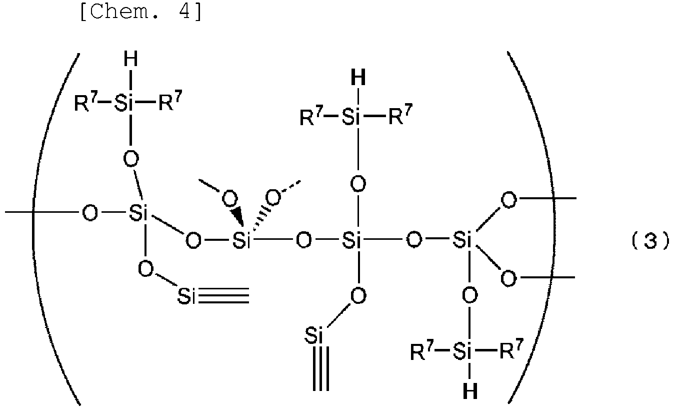

In Formula (3), R⁷ represents a substituted or unsubstituted alkyl group or aryl group having 1 to 8 carbon atoms, a hydrocarbon group as a combination of these, or a hydrogen atom. Examples of the alkyl group having 1 to 8 carbon atoms include a methyl group, an ethyl group, and a propyl group. Among these, a methyl group is preferable. Examples of the aryl group having 1 to 8 carbon atoms include a phenyl group. Examples of a substituent of R⁷ include a methyl group.

In Formula (3), a plurality of R⁷'s may be independent from each other, and may be the same or different from each other.

In addition, in Formula (3), "-O-Si≡" represents that Si has a branched structure which spreads three-dimensionally.

As the branched organohydrogen polysiloxane (B2), only one kind may be used alone, or two or more kinds may be used in combination.

In addition, in each of the linear organohydrogen polysiloxane (B1) and the branched organohydrogen polysiloxane (B2), an amount of hydrogen atoms directly bonded to Si (hydride groups) is not particularly limited. However, in the silicone rubber-based curable composition, the total amount of hydride groups in the linear organohydrogen polysiloxane (B1) and the branched organohydrogen polysiloxane (B2) is preferably 0.5 to 5 mol and more preferably 1 to 3.5 mol with respect to 1 mol of vinyl groups in the vinyl group-containing linear organopolysiloxane (A1). As a result, a crosslinked network can be reliably formed between the linear organohydrogen polysiloxane (B1) and the branched organohydrogen polysiloxane (B2), and the vinyl group-containing linear organopolysiloxane (A1).

### <<Silica Particles (C)>>

The silicone rubber-based curable composition of the present embodiment contains a non-conductive filler. The non-conductive filler may include silica particles (C) as necessary. As a result, hardness and mechanical strength of the elastomer can be improved.

The insulating silicone rubber-based curable composition and the conductive silicone rubber-based curable composition may contain the same type of non-conductive fillers. It is sufficient that the same kind of non-conductive filler may have at least a common constituent material, and the particle diameter, the specific surface area, the surface treatment agent, or the addition amounts may be different.

The insulating silicone rubber-based curable composition and the conductive silicone rubber-based curable composition may further contain different types of silane coupling agents.

The silica particles (C) are not particularly limited. For example, fumed silica, sintered silica, precipitated silica, or the like is used. These may be used alone or in combination of two or more thereof.

A specific surface area of the silica particles (C), which is measured by, for example, a BET method, is, for example, preferably 50 to 400 m²/g and more preferably 100 to 400 m²/g. The average primary particle diameter of the silica particles (C) is, for example, preferably 1 to 100 nm and more preferably approximately 5 to 20 nm.

Using the silica particles (C) having the specific surface area and the average particle diameter in the above ranges makes it possible to improve the hardness and the mechanical strength of the formed silicone rubber and, particularly, to improve the tensile strength of the formed silicone rubber.

### <<Silane Coupling Agent (D)>>

The silicone rubber-based curable composition according to the present embodiment may contain a silane coupling agent (D).

The silane coupling agent (D) may have a hydrolyzable group. The hydrolyzable group is hydrolyzed into a hydroxyl group by water. The hydroxyl group has a dehydration condensation reaction with a hydroxyl group on the surface of the silica particles (C), which can modify the surface of the silica particles (C).

The insulating silicone rubber-based curable composition and the conductive silicone rubber-based curable composition may contain the same kind of silane coupling agent. It is sufficient that the same kind of silane coupling agent has at least a common functional group, and it may have different functional groups in the molecule or different addition amounts thereof.

The insulating silicone rubber-based curable composition and the conductive silicone rubber-based curable composition may further contain different types of silane coupling agents.

In addition, the silane coupling agent (D) may include a silane coupling agent having a hydrophobic group. As a result, the hydrophobic group is added to a surface of the silica particles (C), and in the silicone rubber-based curable composition and in the silicone rubber, it is presumed that a cohesion force between the silica particles (C) decreases (cohesion through a hydrogen bond formed by a silanol group decreases), and thus a dispersibility of the silica particles (C) in the silicone rubber-based curable composition is improved. Consequently, an interface between the silica particles (C) and a rubber matrix enlarges, and a reinforcing effect of the silica particles (C) increases. Furthermore, it is presumed that, when the rubber matrix is deformed, a slipperiness of the silica particles (C) in the matrix is improved. By improving the dispersibility and slipperiness of the silica particles (C), a mechanical strength (for example, a tensile strength, a tear strength, or the like) of the silicone rubber by the silica particles (C) is improved.

Furthermore, the silane coupling agent (D) may include a silane coupling agent having a vinyl group. As a result, the vinyl group is introduced onto the surface of the silica particles (C). Therefore, in a case where the silicone rubber-based curable composition is cured, that is, in a case where the vinyl groups of the vinyl group-containing organopolysiloxane (A) and the hydride groups in the organohydrogen polysiloxane (B) have a hydrosilylation reaction to form a network (crosslinked structure) using this reaction, the vinyl groups of the silica particles (C) are also involved in the hydrosilylation reaction with the hydride groups of the organohydrogen polysiloxane (B). As a result, the silica particles (C) are also incorporated into the network. Accordingly, it is possible to achieve the reduction in hardness and the increase in modulus of the formed silicone rubber.

As the silane coupling agent (D), a silane coupling agent having a hydrophobic group and a silane coupling agent having a vinyl group can be used in combination.

Examples of the silane coupling agent (D) include a compound represented by the following Formula (4).

Yₙ-Si-(X)₄₋ₙ (4)

In Formula (4), n represents an integer of 1 to 3. Y represents any functional group selected from the group consisting of a hydrophobic group, a hydrophilic group, and a vinyl group, in a case where n represents 1, Y represents a hydrophobic group, and in a case where n represents 2 or 3, at least one of Y's represents a hydrophobic group. X represents a hydrolyzable group.

The hydrophobic group is an alkyl group or aryl group having 1 to 6 carbon atoms, or a hydrocarbon group including a combination thereof. Examples thereof include a methyl group, an ethyl group, a propyl group, and a phenyl group; and among these, a methyl group is particularly preferable.

Examples of the hydrophilic group include a hydroxyl group, a sulfonate group, a carboxyl group, a carbonyl group, and the like. Among these, a hydroxyl group is particularly preferable. The hydrophilic group may be included as a functional group, but from the viewpoint of imparting hydrophobicity to the silane coupling agent (D), it is preferable that the hydrophilic group is not included.

Examples of the hydrolyzable group include an alkoxy group such as a methoxy group or an ethoxy group, a chloro group, a silazane group, and the like. Among these, in view of high reactivity with the silica particles (C), a silazane group is preferable. Owing to its structural characteristics, the silane coupling agent having a silazane group as a hydrolyzable group has two structures represented by (Yₙ-Si-) in Formula (4).

Specific examples of the silane coupling agent (D) represented by Formula (4) are as follows.

Examples of the silane coupling agent having a hydrophobic group as the functional group include an alkoxysilane such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, and decyltrimethoxysilane; a chlorosilane such as methyltrichlorosilane, dimethyldichlorosilane, trimethylchlorosilane, and phenyltrichlorosilane; and hexamethyldisilazane. Among these, a silane coupling agent having a trimethylsilyl group, which includes one or more selected from the group consisting of hexamethyldisilazane, trimethylchlorosilane, trimethylmethoxysilane, and trimethylethoxysilane, is preferable.

Examples of the silane coupling agent having a vinyl group as the functional group include an alkoxysilane such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, and vinylmethyldimethoxysilane; a chlorosilane such as vinyltrichlorosilane and vinylmethyldichlorosilane; and divinyltetramethyldisilazane. Among these, a silane coupling agent having a vinyl group-containing organosilyl group, which includes one or more selected from the group consisting of methacryloxypropyltriethoxysilane, methacryloxypropyltrimethoxysilane, methacryloxypropylmethyldiethoxysilane, methacryloxypropylmethyldimethoxysilane, divinyltetramethyldisilazane, vinyltriethoxysilane, vinyltrimethoxysilane, and vinylmethyldimethoxysilane, is preferable.

In addition, in a case where the silane coupling agent (D) includes two kinds including the silane coupling agent having a trimethylsilyl group and the silane coupling agent having a vinyl group-containing organosilyl group, it is preferable that hexamethyldisilazane is included as the silane coupling agent having a hydrophobic group and divinyltetramethyldisilazane is included as the silane coupling agent having a vinyl group.

In a case where a silane coupling agent (D1) having a trimethylsilyl group and a silane coupling agent (D2) having a vinyl group-containing organosilyl group are used in combination, a ratio between (D1) and (D2) is not particularly limited. However, for example, a weight ratio (D1):(D2) is 1:0.001 to 1:0.35, preferably 1:0.01 to 1:0.20, and more preferably 1:0.03 to 1:0.15. In a case where the ratio is set to be in the above-described numerical ranges, desired physical properties of silicone rubber can be obtained. Specifically, a balance between the dispersibility of silica in the rubber and the crosslinkability of the rubber can be achieved.

In the present embodiment, a lower limit value of a content of the silane coupling agent (D) is preferably 1 mass% or more, more preferably 3 mass% or more, and still more preferably 5 mass% or more with respect to 100 parts by weight of the total amount of the vinyl group-containing organopolysiloxane (A). In addition, an upper limit value of the content of the silane coupling agent (D) is preferably 100 mass% or less, more preferably 80 mass% or less, and still more preferably 40 mass% or less with respect to 100 parts by weight of the total amount of the vinyl group-containing organopolysiloxane (A).

By adjusting the content of the silane coupling agent (D) to be the above-described lower limit value or more, adhesiveness between a columnar portion containing an elastomer and a conductive resin layer can be improved. In addition, the improvement of the mechanical strength of the silicone rubber can be promoted. In addition, by adjusting the content of the silane coupling agent (D) to be the above-described upper limit value or less, the silicone rubber can have appropriate mechanical properties.

### <<Platinum or Platinum Compound (E)>>

The silicone rubber-based curable composition according to the present embodiment may contain a catalyst. The catalyst may include platinum or a platinum compound (E). The platinum or platinum compound (E) is a catalyst component which functions as a catalyst during the curing. The addition amount of the platinum or platinum compound (E) is the catalytic amount.

The insulating silicone rubber-based curable composition and the conductive silicone rubber-based curable composition may contain the same type of catalysts. It is sufficient that the same kind of catalyst has at least a common constituent material, and it may have different compositions in the catalyst or different addition amounts thereof.

The insulating silicone rubber-based curable composition and the conductive silicone rubber-based curable composition may further contain different kinds of catalysts.

As the platinum or platinum compound (E), known platinum or platinum compounds can be used. Examples thereof include platinum black, silica or carbon black on which platinum is supported, chloroplatinic acid or an alcohol solution of platinic chloride, a complex salt of platinic chloride and olefin, a complex salt of platinic chloride and vinylsiloxane, and the like.

As the platinum or platinum compound (E), only one kind may be used alone or two or more kinds may be used in combination.

In the present embodiment, a content of the platinum or platinum compound (E) in the silicone rubber-based curable composition refers to the catalytic amount and can be appropriately set. Specifically, a content of platinum-group metal in units of weight is 0.01 to 1000 ppm, preferably 0.1 to 500 ppm, with respect to 100 parts by weight of the total amount of the vinyl group-containing organopolysiloxane (A), the silica particles (C), and the silane coupling agent (D).

By adjusting the content of the platinum or platinum compound (E) to be the above-described lower limit value or more, the silicone rubber-based curable composition can be cured at an appropriate rate. In addition, by adjusting the content of the platinum or platinum compound (E) to be the above-described upper limit value or less, a reduction in manufacturing costs can be promoted.

### <<Water (F)>>

In addition, the silicone rubber-based curable composition according to the present embodiment may contain water (F) in addition to the above-described components (A) to (E).

The water (F) is a component that functions as a dispersion medium for dispersing the respective components in the silicone rubber-based curable composition and contributes to the reaction between the silica particles (C) and the silane coupling agent (D). Therefore, in the silicone rubber, the silica particles (C) and the silane coupling agent (D) can be more reliably bonded to each other, and uniform properties can be exhibited as a whole.

### (Other Components)

Furthermore, the silicone rubber-based curable composition according to the present embodiment may further contain other components in addition to the above-described components (A) to (F). Examples of the other components include an inorganic filler other than the silica particles (C), such as diatomaceous earth, iron oxide, zinc oxide, titanium oxide, barium oxide, magnesium oxide, cerium oxide, calcium carbonate, magnesium carbonate, zinc carbonate, glass wool, and mica; and an additive such as a reaction inhibitor, a dispersant, a pigment, a dye, an antistatic agent, an antioxidant, a flame retardant, and a thermal conductivity enhancing agent.

The conductive solution (conductive silicone rubber composition) according to the present embodiment contains the above-described conductive filler and a solvent, in addition to the above-described silicone rubber-based curable composition not containing the conductive filler.

As the above-described solvent, various well-known solvents can be used, and for example, a high boiling point solvent can be included. These may be used alone or in combination of two or more thereof.

Examples of the solvent include an aliphatic hydrocarbon such as pentane, hexane, cyclohexane, heptane, methylcyclohexane, ethylcyclohexane, octane, decane, dodecane, and tetradecane; an aromatic hydrocarbon such as benzene, toluene, ethylbenzene, xylene, trifluoromethylbenzene, and benzotrifluoride; an ether such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, and tetrahydrofuran; a haloalkane such as dichloromethane, chloroform, 1,1-dichloroethane, 1,2-dichloroethane, 1,1,1-trichloroethane, and 1,1,2-trichloroethane; a carboxylic acid amide such as N,N-dimethylformamide and N,N-dimethylacetamide; and a sulfoxide such as dimethyl sulfoxide and diethyl sulfoxide. These may be used alone or in combination of two or more thereof.

By adjusting the solid content in the solution, the above-described conductive solution can have an appropriate viscosity for various coating methods such as spray coating and dip coating.

In addition, in a case where the above-described conductive solution contains the above-described conductive filler and the above-described silica particles (C), a lower limit value of a content of the silica particles (C) in the electrode portion main body 39 is, for example, 1 mass% or more, preferably 3 mass% or more, and more preferably 5 mass% or more, with respect to 100 mass% of the total amount of the silica particles (C) and the conductive filler. As a result, the mechanical strength of the electrode portion main body 39 can be improved. On the other hand, an upper limit value of the above-described content of the silica particles (C) in the electrode portion main body 39 is, for example, 20 mass% or less, preferably 15 mass% or less, and more preferably 10 mass% or less, with respect to 100 mass% of the total amount of the silica particles (C) and the conductive filler. As a result, a balance between the conductivity and the mechanical strength or flexibility in the electrode portion main body 39 can be achieved.

By heating and drying the conductive solution as necessary, the conductive silicone rubber is obtained.

The conductive silicone rubber may be configured not to contain a silicone oil. As a result, a decrease in electrical continuity due to bleeding out the silicone oil to a surface of the electrode portion main body 39 can be suppressed.

### <Material for Conductive Contact Portion>

The conductive member of the conductive contact portion 33 is, for example, a paste containing a highly conductive metal (so-called conductive paste). The highly conductive metal includes one or more selected from the group consisting of copper, silver, gold, nickel, tin, lead, zinc, bismuth, antimony, and an alloy thereof. In particular, from the viewpoint of availability and conductivity, silver, silver chloride, or copper is preferable.

In a case where the conductive contact portion 33 is formed with the paste containing a highly conductive metal, an apex portion of the protrusion portion 32 made of the rubber-like elastic body is dipped (dip-coated) in a paste-like conductive solution containing the highly conductive metal. As a result, the conductive contact portion 33 is formed on the surface of the protrusion portion 32.

The conductive contact portion 33 as a conductive resin layer may be formed by applying the conductive solution containing the conductive filler and the solvent onto the protrusion portion 32. In this case, the solvent is based on the same material (silicone rubber) as the material for the protrusion portion 32, so that the adhesiveness of the conductive contact portion 33 (conductive resin layer) can be enhanced.

By heating and drying the conductive solution as necessary, the conductive silicone rubber is obtained.

The conductive silicone rubber may be configured not to contain a silicone oil. As a result, a decrease in electrical continuity due to the silicone oil bleeding out to a surface of the conductive contact portion 33 can be suppressed.

As a result, ability to part hair when the brain wave measurement device 10 is mounted on the head 99 can be improved. In addition, a sufficient contact area of the conductive contact portion 33 at the time of mounting the brain wave measurement device 10 can be ensured.

### <Structure of Signal Line Portion>

The electrode unit 30 is provided with a signal line portion 34 as a signal path connected to the conductive contact portion 33. Various wiring structures can be adopted for the signal line portion 34 as long as the signal line portion 34 is in a state of being conducted through the base portion 31 and the protrusion portion 32. Here, the signal line portion 34 is provided to pass through the inside of the protrusion portion 32 and the base portion 31 from the conductive contact portion 33 at the tip of the protrusion portion 32 and to be exposed on the base portion upper surface 37. A portion (here, an end portion 34a) of the signal line portion 34 which protrudes from the base portion upper surface 37 is interposed between the male snap button 35 (more specifically, a disk portion 35a described later) and the base portion upper surface 37, and electrical connection with the male snap button 35 is ensured.

The lower-side tip of the signal line portion 34 may have any of a protruding structure, a substantially coplanar structure, or a buried structure with respect to a distal end portion of the protrusion portion 32 or the vicinity thereof, that is, the region where the conductive contact portion 33 is formed. From the viewpoint of connection stability with the conductive contact portion 33, a protruding structure may be used. A protruding portion at the distal end of the signal line portion 34 is partially or entirely covered with the conductive contact portion 33. For the protruding structure at the distal end of the signal line portion 34, it is possible to adopt a structure with or without folds or a structure wrapped around the surface of the distal end portion of the protrusion portion 32.

As another wiring structure of the signal line portion 34, a structure provided on the surfaces of the protrusion portion 32 and the base portion 31 may be used, or a wiring structure in which a part is provided on the inside and a part is provided on the surface may be used. That is, a signal detected by the conductive contact portion 33 may be finally transmitted to the male snap button 35.

### <Material for Signal Line Portion>

As the signal line portion 34, known materials can be used. For example, the signal line portion 34 can be configured with conductive fiber. As the conductive fiber, it is possible to use one or more fibers selected from the group consisting of metal fiber, metal-coated fiber, carbon fiber, conductive polymer fiber, conductive polymer-coated fiber, and conductive paste-coated fiber. These may be used alone or in combination of two or more thereof.

The metal material for the metal fiber and the metal-coated fiber is not limited as long as it has conductivity. Examples of the metal material include copper, silver, gold, nickel, tin, lead, zinc, bismuth, antimony, stainless steel, aluminum, silver/silver chloride, and alloys of these. These may be used alone or in combination of two or more thereof. Among these, from the viewpoint of electrical continuity, silver can be used. In addition, it is preferable that the metal material does not include a metal such as chromium, which imposes a burden on the environment.

The fiber material for the metal-coated fiber, the conductive polymer-coated fiber, or the conductive paste-coated fiber described above is not particularly limited and may be any one of synthetic fiber, semisynthetic fiber, or natural fiber. Among these, for example, polyester, nylon, polyurethane, silk, or cotton is preferably used. These may be used alone or in combination of two or more thereof.

Examples of the above-described carbon fiber include a PAN-based carbon fiber and a pitch-based carbon fiber.

As the conductive polymer material for the conductive polymer fiber and the conductive polymer-coated fiber, for example, a mixture of a conductive polymer such as polythiophene, polypyrrole, polyaniline, polyacetylene, polyphenylene vinylene, polynaphthalene, or a derivative of these, and a binder resin, or an aqueous solution of a conductive polymer such as PEDOT-PSS ((3,4-ethylenedioxythiophene)-poly(styrene sulfonate)) is used.

The resin material contained in the conductive paste of the conductive paste-coated fiber is not particularly limited, and preferably has stretchability. For example, the resin material includes one or more materials selected from the group consisting of silicone rubber, urethane rubber, fluororubber, nitrile rubber, acrylic rubber, styrene rubber, chloroprene rubber, and ethylene propylene rubber. These may be used alone or in combination of two or more thereof.

A conductive filler in the conductive paste of the conductive paste-coated fiber described above is not particularly limited, and a well-known conductive material may be used. For example, the conductive filler may include one or more selected from the group consisting of metal particles, metal fiber, metal-coated fiber, carbon black, acetylene black, graphite, carbon fiber, carbon nanotube, a conductive polymer, conductive polymer-coated fiber, and metal nanowire.

A metal forming the above-described conductive filler is not particularly limited. For example, the metal may include at least one or two or more among copper, silver, gold, nickel, tin, lead, zinc, bismuth, antimony, silver/silver chloride, and alloys thereof. Among these, from the viewpoint of high conductivity or high availability, silver or copper is preferable.

The signal line portion 34 may be formed of twisted yarn obtained by twisting a plurality of linear conductive fibers. As a result, disconnection of the signal line portion 34 during deformation can be suppressed.

In the present embodiment, the coating of the conductive fiber includes not only that the outer surface of the fiber material is covered with the conductive material, but also that gaps between fibers in twisted yarn obtained by twisting single fibers are impregnated with metal, the conductive polymer, or the conductive paste such that each of the single fibers forming the twisted yarn is coated individually.

A tensile elongation at break of the signal line portion 34 is, for example, 1% or more and 50% or less, preferably 1.5% or more and 45%. In such a numerical range, excessive deformation of the protrusion portion 32 can be suppressed while suppressing break during deformation.

### <Support Member>

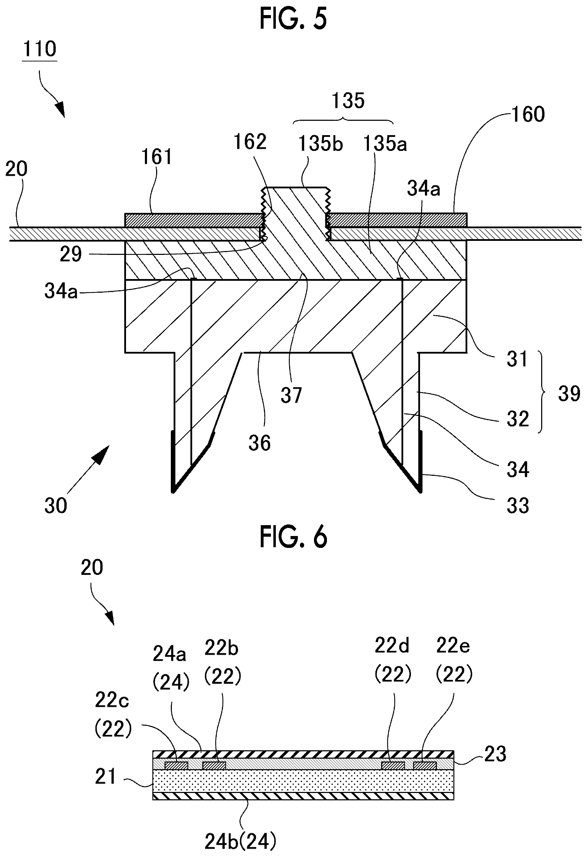

The support member 20 will be specifically described mainly with reference to Figs. 3 and 6. Fig. 3 is a plan view schematically showing the base material 21 and the circuit pattern 22 in the support member 20. The shield layer 24 is omitted in the drawing. Fig. 6 is a cross-sectional view of the support member 20, and here shows a cross-sectional view taken along a line X2-X2 in Fig. 2.

The support member 20 has a so-called flexible substrate and is provided in an elongated shape. The support member 20 is configured to have an elongated base material 21, the circuit pattern 22, the protective layer 23, the shield layer 24, and a cord guide portion 26.

### <Base Material>

The base material 21 is provided with the mounting portions 25 at five positions corresponding to the positions T3, C3, Cz, C4, and T4 in the international 10-20 electrode placement method.

The base material 21 is non-stretchable, has flexibility in a direction perpendicular to a surface formed by the base material 21, and is configured to have a sufficient strength for attaching the electrode unit 30. That is, the base material 21 has a sufficient strength such that it is not damaged when the mounting portion 25 is provided and the electrode unit 30 is attached. The non-stretchability is defined by a Poisson's ratio described later.

In addition, from the viewpoint that the plurality of electrode units 30 are pressed against the head 99 with an appropriate pressure, that is, they become linear between the mounting portions 25 and a constant tension acts thereon, it has non-stretchable physical properties.

A vertical width (length in a depth direction) of the base material 21 depends on the size of the electrode unit 30 to be mounted, but can be set to, for example, 10 mm to 50 mm.

A horizontal width (length in a left-right direction) of the base material 21 depends on the size of the head 99 and the electrode position, but can be set to, for example, 200 mm to 400 mm. In addition, a distance between the electrodes can be set to, for example, 65 mm to 75 mm assuming an adult use.

A thickness of the base material 21 depends on the material, but can be set to, for example, 0.01 mm to 1 mm. By setting the thickness of the support member 20 to be within the above-described range, it is possible to appropriately follow the shape of the head 99, and to bring the electrode unit 30 into a state of being pressed with a constant tension. The base material 21 may be bent to an extent that the tension does not substantially change depending on the shape of the head 99 or the state of the head hair. In addition, the support member 20 has non-stretchable physical properties from the viewpoint of pressing the plurality of electrode units 30 against the head 99 with an appropriate pressure, that is, applying a constant tension.

As the base material 21, a resin film base material, a metal film member, or a glass film can be used. Examples of the resin film base material include a polyimide resin-based film such as a polyimide resin film, a polyetherimide resin film, and a polyamide-imide resin film; a polyamide resin-based film such as a polyamide resin film; a polyester resin-based film such as a polyester resin film; a PET (polyethylene terephthalate) resin-based film; and a PS (polystyrene) resin-based film. Among the above, from the viewpoint of improving flexibility, elastic modulus, and heat resistance, a polyimide resin-based film is particularly preferably used. In addition, as the metal member, for example, an aluminum foil or a copper foil can be used.

### <Circuit Pattern>

The circuit pattern 22 has, for example, a terminal electrically connected to the electrode unit 30 and a wiring connecting the electrode unit 30 and an external connection terminal (not shown). For example, the circuit pattern 22 is obtained by patterning a flexible copper-clad laminate in which a copper foil is provided on the entire upper surface of the base material 21 into a desired shape by etching. In addition, the method for providing the circuit pattern 22 is not limited to the above-described method, and for example, a method of printing a desired pattern using a conductive paste such as a silver ink and a copper ink can also be used.

The circuit pattern 22 has first to fifth circuit patterns 22a to 22e (simply referred to as the circuit pattern 22 when not distinguished) provided to extend leftward from each of five openings 29. The first to fifth circuit patterns 22a to 22e are independent of each other and electrically insulated from each other.

Each circuit pattern 22 has an annular pattern 28a provided in an annular shape on a peripheral edge of the opening 29, and a linear pattern 22b extending linearly leftward in the drawing from the annular pattern 28a. The mounting portion 25 formed of an annular highly conductive material is attached to the annular pattern 28a, whereby electrical connection between the circuit pattern 22 and the mounting portion 25 is established. That is, a part of the annular pattern 28a functions as a connection terminal with the electrode unit 30. In addition, another part of the linear pattern 22b functions as a wiring to the outside.

The linear pattern 22b partially changes its direction to an oblique direction so as to be separated from another linear pattern 22b or the annular pattern 28a. In addition, since the adjustment part 50 is sewn between the electrode positions C3 and T3 and between the electrode positions C4 and T4, the circuit pattern 22 (annular pattern 28a) is formed avoiding a region necessary for sewing. For example, a connector (not shown) for connection to an external wiring is provided at an end portion of the linear pattern 28b (the left end portion in the drawing).

As the circuit pattern 22, from the viewpoint of imparting a predetermined strength to the support member 20, not only an actual wiring structure but also a dummy pattern may be provided.

### <Protective Layer>

As shown in Fig. 6, the protective layer 23 is a layer in which the periphery (excluding a lower surface) of the circuit pattern 22 is covered with an insulating member, and may be, for example, a coverlay film composed of an insulating resin film and an adhesive, or may be formed by forming a liquid resin composition containing a thermosetting resin by a screen printing method or the like and curing by heating.

### <Shield Layer>

The shield layer 24 is an electromagnetic wave shielding member which covers the circuit pattern 22. The shield layer 24 is provided as a countermeasure against noise when transmitting a brain wave signal through the circuit pattern 22, and has a first shield layer 24a provided on an upper surface of the protective layer 23 and a second shield layer 24b provided on a lower surface of the base material 21. The first shield layer 24a and the second shield layer 24b may be formed of the same material or may be formed of different materials.

The shield layer 24 (the first shield layer 24a and the second shield layer 24b) is obtained by forming a layer serving as an electromagnetic wave shield on the protective layer 23 using a method such as screen printing of a conductive paste containing a metal filler. As the metal filler, a metal such as gold, silver, copper, and aluminum, or an alloy is used alone or in a mixture of a plurality of types. A size of the filler is not particularly limited, but a size of several nm to several µm is used. As components of the paste, the metal filler and a thermosetting epoxy resin or the like are mixed and used.

A thickness of the shield layer 24 (the first shield layer 24a and the second shield layer 24b) can be set to, for example, 5 µm to 50 µm. By setting the thickness to be within the range, a stable thickness can be realized when forming the shield layer 24, and desired shielding performance can be achieved.

As the shield layer 24, a laminate in which a conductive metal layer is formed on a resin film by vapor deposition or the like may be used. In that case, the laminate is bonded to the upper surface of the protective layer 23 or the lower surface of the base material 21 with an adhesive or the like. Although the shield layer may be formed by printing, vapor deposition is preferable from the viewpoint of making the shield layer thin and strongly resistant to bending. In addition, the conductive metal layer is connected to a ground (a portion stabilized at a reference potential) of the circuit pattern 22. Both or one of the first shield layer 24a and the second shield layer 24b may be omitted. In this case, anti-noise performance is deteriorated, but the device can be simplified. In addition, as for the anti-noise performance, a certain range can also be dealt with by signal processing.

### <Physical Property Values and the like of Support Member>

Physical property values (Poisson's ratio, Young's modulus, maximum thickness, Young's modulus × thickness) of the support member 20 (more specifically, the base material 21) are defined, for example, as follows.

The Poisson's ratio and the Young's modulus are measured by a tensile test in accordance with JIS K 7127. In the tensile test, a test piece specified by the above-described standard (a test piece of the base material 21 itself) is used. Although it does not comply with the above-described JIS standard, a test piece in a state in which the circuit pattern 22 is provided on the base material 21 may be used.

### (Poisson's Ratio)

The Poisson's ratio defines the non-stretchability of the support member 20.

A Poisson's ratio of the support member 20 is 0.15 to 0.4. The lower limit of the Poisson's ratio is preferably 0.2 or more, and more preferably 0.25 or more. The upper limit of the Poisson's ratio is preferably 0.38 or less, and more preferably 0.35 or less.

By setting the Poisson's ratio of the support member 20 (that is, the base material 21) to be within the above-described range, the support member 20 is difficult to deform. That is, when the brain wave measurement device 10 is mounted on the head 99, a force acts in a direction in which the support member 20 extends while the electrode unit 30 is pressed against the head 99. That is, a tension acts. In this case, when the support member 20 is an elastic member such as rubber (a member having a Poisson's ratio of approximately 0.46 to 0.49), the support member 20 inappropriately extends, and the force with which the electrode unit 30 presses the head 99 may become non-uniform. As a result, signal quality of the obtained brain waves may be deteriorated. However, by setting the Poisson's ratio of the support member 20 to be within the above-described range, the force with which the electrode unit 30 presses the head 99 can be controlled to be in a certain range, and thus the brain wave measurement can be performed stably.

### (Young's Modulus)

A Young's modulus (elastic modulus) of the support member 20 is 0.4 GPa to 150 GPa. The lower limit value of the Young's modulus is preferably 3 GPa or more, and more preferably 5 GPa or more. The upper limit value thereof is preferably 140 GPa or less and more preferably 135 GPa or less.

By setting the Young's modulus of the support member 20 to be within the above-described range, the strength of the support member 20 can be maintained, and thus deformation can be prevented.

### (Young's Modulus × Thickness)

The product of the Young's modulus (elastic modulus) and the thickness of the material constituting the film member is 0.4 to 9.1.

Even in a case where the material is easily deformable (that is, the material has a small Young's modulus), as the thickness is equal to or more than a certain degree, the force with which the electrode unit 30 presses the head 99 can be controlled within a certain range without substantially deforming the material. In addition, in the case of a hard material (that is, a material having a large Young's modulus), unless the thickness is reduced to some extent, characteristics of causing the brain wave measurement device 10 to follow the shape of the head 99 are significantly deteriorated. Therefore, by setting the product of the Young's modulus (elastic modulus) and the thickness within the above-described range, the force with which the electrode unit 30 attached to the support member 20 presses the head 99 can be controlled within a certain range, and thus stable measurement of the brain waves can be achieved.

### <Connection Structure of Electrode Unit and Support Member>

For example, as shown in Fig. 4, the electrode unit 30 and the support member 20 are fixed by snap button fitting. Specifically, the female snap button 25a of the support member 20 and the male snap button 35 of the electrode unit 30 are configured to be fitted to each other. The female snap button 25a functions as the mounting portion 25 for attaching the electrode unit 30 through a fitting structure.

The male snap button 35 is made of, for example, a highly conductive metal, and has a disk-shaped disk portion 35a and a convex button-shaped button portion 35b which extends from the center of the upper surface of the disk portion 35a. As the highly conductive metal, for example, stainless steel, a copper alloy, an aluminum alloy, brass, or the like can be used.

The disk portion 35a is attached to the base portion upper surface 37 of the base portion 31 with a conductive adhesive or the like. In this case, as described above, the end portion 34a of the signal line portion 34 is interposed between the disk portion 35a and the base portion upper surface 37, and thus the electrical connection with the male snap button 35 is ensured.

The button portion 35b is attached so as to be fitted to the female snap button 25a provided on the support member 20.

The female snap button 25a is formed of a highly conductive metal similarly to the male snap button 35, and outputs the brain wave signal acquired by the electrode unit 30 to the brain wave display device or the like by the circuit pattern 22.

With reference to Fig. 5, a structural example adopting screw fitting instead of the snap button fitting as a modification example of the fixing structure of the electrode unit 30 and the support member 20 will be described.

In the modification example, a male screw connection terminal 135 provided at the top of the electrode unit 30, and an electrode attachment part 160 provided instead of the female snap button 25a as the mounting portion 25 are included.

The male screw connection terminal 135 is made of, for example, a highly conductive metal, and has a disk-shaped disk portion 135a and a protruding portion 135b extending from the center of an upper surface of the disk portion 135a. The protruding portion 135b is formed in a cylindrical shape, and a male screw is formed on a peripheral surface thereof. As the highly conductive metal, for example, stainless steel, a copper alloy, an aluminum alloy, brass, or the like can be used.

The disk portion 135a is attached to the base portion upper surface 37 of the base portion 31 with a conductive adhesive or the like. At this time, as described above, the end portion 34a of the signal line portion 34 is interposed between the disk portion 135a and the base portion upper surface 37, and thus electrical conduction with the male screw connection terminal 135 is ensured.

The electrode attachment part 160 is formed of a highly conductive material, and has an attachment part main body 161 which is circular in a top view, and a female screw connection terminal 162 provided at the center of the attachment part main body 161. The female screw connection terminal 162 is a threaded opening into which the male screw connection terminal 135 (protruding portion 135b) of the electrode unit 30 is screwed. As a result, the fixing can be performed more firmly than the snap button fitting, and occurrence of noise caused by the fitting portion can be suppressed.

An outer diameter of the attachment part main body 161 is set to be substantially the same as the outer diameter of the electrode unit 30. An upper surface of the attachment part main body 161 is formed of a hard insulating substrate, on which a circuit such as an amplifier unit (preamplifier) that primarily amplifies the brain waves acquired by the electrode unit 30 is mounted. The upper surface of the attachment part main body 161 is covered with a cover member such as the shield layer 24 as necessary.

The male screw connection terminal 135 protruding from the female screw connection terminal 162 may be fixed to the attachment part main body 161 with a nut (not shown). As a result, the fixing of the electrode unit 30 and the support member 20 becomes firmer, and transmission of the brain wave signal is further stabilized.

### <Attachment Part>

As shown in Figs. 1 and 2, the attachment part 70 is a member attached to a measurement site (for example, an ear) of a subject in order to make a mounting state appropriate when mounting the brain wave measurement device 10 on the head 99 of the subject. In other words, the attachment part 70 is provided at an end portion of the support member 20, is attached to an ear or a jaw of the measurement subject, and functions as an assisting member for assisting placement of the support member 20 along the shape of the head 99.

The attachment part 70 is attached to each of both ends in a longitudinal direction (both ends in a left-right direction in Figs. 1 and 2) of the support member 20, and spans between a site (here, an ear) different from the measurement site of the subject and the support member 20. Accordingly, the electrode unit 30 is pressed against the head 99 with a predetermined electrode pressing force F.

Specifically, the attachment part 70 includes an adjustment part 50, a tension display portion 60, an ear attachment portion 40, a non-stretchable cord 55 that connects the adjustment part 50 and the tension display portion 60, and a cord 45 that connects the tension display portion 60 and the ear attachment portion 40.

### <Ear Attachment Portion>

The ear attachment portion 40 is mounted on the ear of the subject. The cord 45 is attached to the ear attachment portion 40, and is attachably and detachably attached to a second hook 64 of the tension display portion 60 by a ring 46 provided at an end portion of the cord 45. In the example of the present embodiment, the ear attachment portion 40 is mounted such that the cord 45 wraps around from below the ear.

### <Adjustment Part>

The adjustment part 50 adjusts the tension acting on the cord 55 by adjusting the distance (length) between the support member 20 and the ear attachment portion 40.

The adjustment part 50 is attached to each of both ends of the support member 20. The adjustment part 50 includes a plate-shaped member 52 that is attached to the support member 20, a locking portion 51 for fixing a length of the plate-shaped member 52, a cord 55 that is attached to an end portion of the plate-shaped member 52 on the tension display portion 60 side, and a ring 56 that is provided at an end portion of the cord 55 on the tension display portion 60 side. A first hook 63 of the tension display portion 60 is attachably and detachably attached to the ring 56.

The plate-shaped member 52 has a long strip shape in which a plurality of teeth are arranged in a row. The locking portion 51 is provided with an opening in which a claw is formed, and the plate-shaped member 52 is inserted into the opening and locked at a desired position. In addition, the locking portion 51 is provided with a release portion which releases the locked state in conjunction with the claw. A material for the adjustment part 50 is not particularly limited, and various plastics can be used. From the viewpoints of physical properties, processability, cost, and the like, nylon 66 can be preferably used.

### <Tension Display Portion>

The tension display portion 60 is attachably and detachably provided between the adjustment part 50 and the ear attachment portion 40, and displays whether or not a tension acting on both ends thereof is within an appropriate range. That is, it is possible to determine whether or not the tension T1 applied to the end portion of the support member 20 (film base material) is within an appropriate predetermined range in the brain wave measurement.

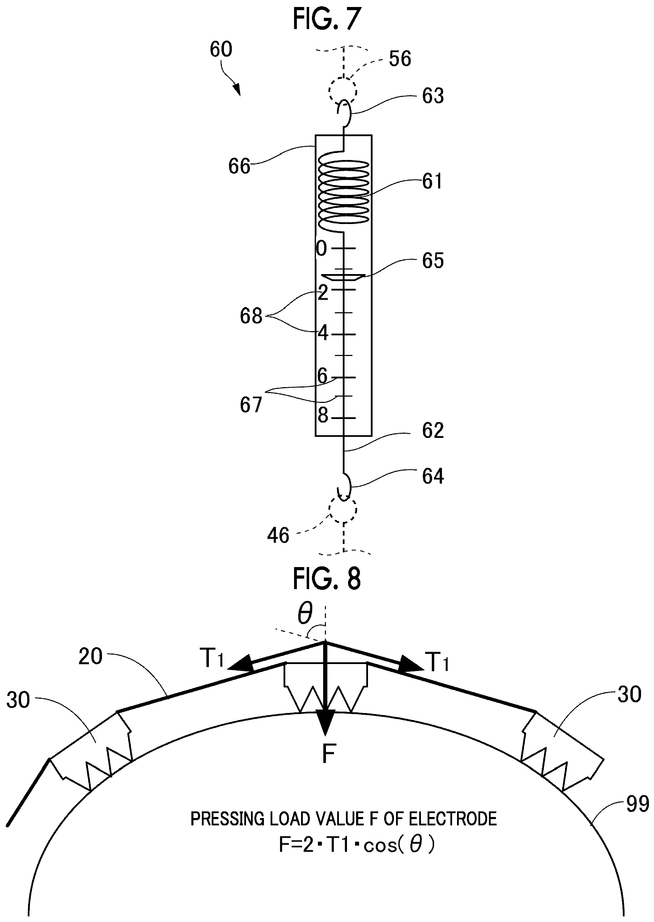

As shown in Fig. 7, the tension display portion 60 has a cylindrical housing 66, a stretchable elastic body (spring 61) housed inside the housing 66, and an indicator 65, a scale 67, and an indicator section 68 indicating an extended state of the spring 61. The elastic body (spring 61) indicates the tension acting on the support member 20 in a recognizable manner.

The housing 66 is configured to be able to determine at least a position of the indicator 65. In the present embodiment, the housing 66 is transparent such that an internal state is recognizable. One end portion (here, an end portion on the adjustment part 50 side) of the housing 66 is closed, and the other end portion (here, an end portion on the ear attachment portion 40 side) is an opening. The first hook 63 is provided at an upper end portion of the housing 66, and is attachably and detachably attached to the ring 56 provided at a distal end of the cord 55 of the adjustment part 50.

The spring 61 is, for example, a coil spring. One end portion (end portion on the adjustment part 50 side) of the spring 61 is fixed to a closed end portion (end portion on the adjustment part 50 side) of the housing 66. The spring 61 is provided so as to extend to a certain degree when the brain wave measurement device 10 is mounted on the head 99. The degree of extension is set to such an extent that the extended state can be visually grasped at the indicator 65 described later and to such an extent that an excessive force does not act on the head 99.

A non-stretchable wire 62 is attached to the other end portion (end portion on the ear attachment portion 40 side) of the spring 61, and extends from an end portion of the housing 66 on the opening side to the outside. The second hook 64 is provided at a distal end of the wire 62. The second hook 64 is attachably and detachably attached to the ring 46 provided at an end portion of the cord 45 of the ear attachment portion 40.

The indicator 65 is provided at an attachment portion of the spring 61 and the wire 62. The indicator 65 indicates an extension of the spring 61. The scale 67 and the indicator section 68 are provided on a peripheral surface of the housing 66. The scale 67 is provided with a plurality of horizontal lines at a constant interval. The indicator section 68 is provided as a numerical value that can be determined for the tension. The indicator 65, the scale 67, and the indicator section 68 function as a proper tension indicating portion that displays whether or not the tension is within an appropriate range in a recognizable manner. The scale 67 may include a mark or the like, indicating the proper range with a red line or the like so that it can be easily recognized that it is within the proper range.

It is also assumed that an appropriate tension varies depending on the attribute of the subject (for example, age, gender, and state of head hair). Therefore, a plurality of types of tension display portions 60 having different strengths of the spring 61, ranges of the scale 67 and the indicator section 68, and the like may be prepared, and an appropriate tension display portion 60 may be selected according to the subject.

### <Electrode Pressing Force F on Head>

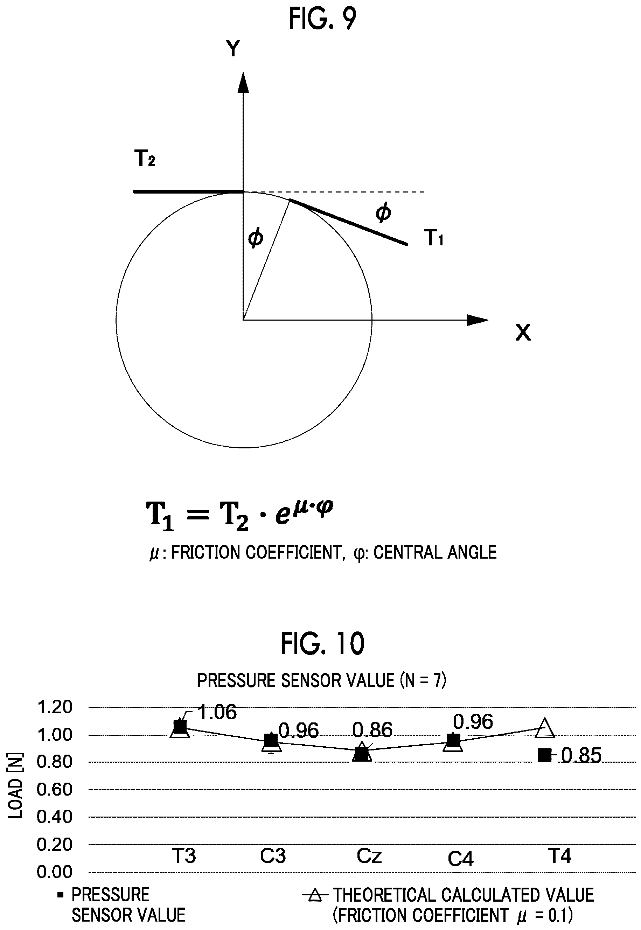

Features of the present embodiment will be described with reference to Figs. 8 and 9. Fig. 8 is a view for explaining, based on a model, a force acting on the head 99 (electrode pressing force F to the head) when the brain wave measurement device 10 is mounted. Fig. 9 is a view for explaining the capstan principle (capstan equation) used for modeling.

As shown in Fig. 8, in a state in which the brain wave measurement device 10 is mounted on the head 99, a polygon (or a polygonal line) is formed in which a plurality of electrode units 30 serve as vertices and the electrode units 30 are connected by the support member 20. In this case, assuming that the electrode unit 30 is pressed against the head 99 by a tension of the support member 20, a force (electrode pressing force F) for pressing the electrode unit 30 against the head 99 can be represented by the following expression 1. $F = 2 ⋅ {T}_{1} ⋅ cos \left(θ\right)$ Expression 1

That is, a difference in θ caused by individual difference in unevenness of the head appears as the difference in the electrode pressing force F.

Furthermore, the minimum curvature of an inscribed circle is obtained from a height and an interval of the electrode units 30. In addition, the maximum curvature is obtained from the capstan principle with the electrode pressing force F required for the electrode unit 30. Since the electrode pressing force F on the head 99 follows the capstan equation, the force is larger on a temporal region than on a parietal region.

Here, it is assumed that the support member 20 (film base material) is in a state of being wound around the head 99. Since there is friction between the head 99 and the support member 20, it is considered that the tension of each electrode unit 30 follows the capstan equation shown in the following expression 2. ${T}_{1} = {T}_{2} ⋅ {e}^{∧} \left(μ⋅φ\right)$ Expression 2
- µ:: friction coefficient
- φ:: central angle

From Equation 1 and Equation 2, a relational expression between a tension T1 applied to the end portion of the support member 20 (film base material) and a load (electrode pressing force F) applied to the head 99 by the electrode unit 30 can be grasped.

The load value calculated from the above-described theoretical calculation and a load value actually measured will be shown in Examples (see Fig. 10) described later.

As described above, according to the present embodiment, in the brain wave measurement device 10, by providing the circuit pattern 22 on the support member 20, electric wires or the like can be omitted, and the wiring structure can be simplified while appropriately bringing the electrode unit 30 of the brain wave measurement device 10 into contact with the scalp.

In addition, the electrode units 30 are fixed to the support member 20 formed of the film base material, and the polygon (or the polygonal line) is formed with the electrode units 30 attached only to necessary portions as vertices. Therefore, followability of the electrode unit 30 to the head shape can be improved. In addition, since the mounting state of the brain wave measurement device 10 is modeled by the capstan equation, the electrode pressing force F by the electrode unit 30 can be appropriately grasped.

In addition, by providing the attachment part 70 with the adjustment part 50 and the tension display portion 60, the tension acting on the support member 20 can be adjusted within a proper range. Furthermore, by making the tension display portion 60 attachable and detachable, an appropriate tension display portion 60 can be selected and used according to the attributes of the subject, and stable brain wave measurement can be realized.

More specifically, when all of the support member 20 and the attachment part 70 are non-stretchable members, the tension directly changes when the length is changed by the adjustment part 50. Since the appropriate range of the force with which the electrode unit 30 is pressed against the scalp is narrow, that is, the appropriate range of the adjustment part 50 is also narrow, there is a tendency that the adjustment is difficult. More specifically, the deformation and elasticity of the ear or the scalp have an effect of slightly expanding the appropriate range of the adjustment part 50, but the adjustment is still difficult, the adjustment range is narrow between a loose state with small tension and a painful state with large tension, and it is difficult to mount the brain wave measurement device 10 by pressing the electrode unit 30 with sufficient force without giving an uncomfortable feeling to the subject. On the other hand, in a case where the tension display portion 60 is provided, that is, in a case where a stretchable member is included, not only the above-mentioned deformation and elasticity of the ear or the scalp but also deformation of an elastic body such as a spring having more stretchability are added, whereby the appropriate range of the adjustment part 50 can be largely expanded, so that the mounting of the brain wave measurement device 10 in an appropriate state becomes easy.

The plate-shaped member 152 is a rail-like long strip-shaped member in which a plurality of protruding portions 152a are arranged in a row. The locking portion 151 is slidably joined to a rail formed by the protruding portions 152a. The locking portion 151 has a locking mechanism 156 holding in a non-slidable manner. The non-slidable state is released by a predetermined operation (for example, an operation of pushing in the horizontal direction) with respect to the locking mechanism 156.

By sliding the locking portion 151 on the plate-shaped member 152, the distance between the tension display portion 60 (that is, the ear attachment portion 40) attached to the cord 55 and the support member 20, that is, the tension can be adjusted.

### <Summary of Embodiments>

The features of the embodiment are summarized as follows.
(1) A brain wave measurement device 10 including:
   an electrode unit 30 configured to contact a measurement site on a head 99 of a subject and acquire a brain wave signal;
   a support member 20 configured to support the electrode unit 30; and
   an attachment part 70 (assisting member) configured to assist placement of the support member 20 on the head 99,
   in which the attachment part 70 (assisting member) has a non-stretchable film member and a mounting portion 25 to which the electrode unit 30 is electrically connected and fixed,
   the film member has a non-stretchable base material 21 and a circuit pattern 22 provided on the base material 21, and
   the circuit pattern 22 is connected to the mounting portion 25.
(2) The brain wave measurement device 10 according to (1), further including: an electromagnetic wave shielding member (shield layer 24) configured to cover the circuit pattern 22.
(3) The brain wave measurement device 10 according to (1) or (2),
   in which the film member has an elongated shape, and
   a plurality of electrode units 30 are arranged at predetermined intervals in a longitudinal direction of the film member.
(4) The brain wave measurement device 10 according to any one of (1) to (3), in which the electrode unit 30 has a base portion 31, a plurality of protrusion portions 32 (protruding portions) provided on the base portion 31, and a conductive contact portion 33 (electrode portion) provided on the protrusion portion 32 (protruding portion) and in contact with the head 99.
(5) The brain wave measurement device 10 according to (4), in which the protrusion portions 32 (protruding portions) are elastic members.
(6) The brain wave measurement device 10 according to any one of (1) to (5), in which the attachment part 70 (assisting member) is provided at an end portion of the support member 20, is configured to be attached to an ear or a jaw, and assists placement of the support member 20 along a shape of the head 99.
(7) The brain wave measurement device 10 according to any one of (1) to (6), in which the attachment part 70 (assisting member) includes a member having spring properties.
(8) The brain wave measurement device 10 according to any one of (1) to (7), in which the attachment part 70 (assisting member) includes a member for adjusting a length.
(9) The brain wave measurement device 10 according to any one of (1) to (8), in which the support member 20 is bent at a vertex and linearly connects the vertices, each attachment position (mounting portion 25) of the electrode unit 30 serving as the vertex.
(10) A brain wave measurement method including: mounting the brain wave measurement device 10 according to any one of (1) to (9) on a head 99 of a subject and measuring brain waves.

The embodiments of the present invention have been described above, but these are examples of the present invention and various configurations other than the above can be adopted.

### EXAMPLES

Hereinafter, the present embodiments will be described in detail with reference to Examples. The present embodiments are not limited to the description of Examples.

In Examples, corresponding to the first and second embodiments, "Verification of support member (film base material)" and "Verification of modeling based on Capstan equation" were confirmed.

### <<Verification of Support Member (Film Base Material)>>

Seven samples of Examples 1 to 7 were evaluated as to whether or not they were appropriate as the support member 20.

The evaluation standards were in three stages as follows.
Evaluation A ... deviation of the electrode position was small (5 mm or less) in a case where a constant tension (0.6 N) was applied.
Evaluation B ... deviation of the electrode position was within an allowable range (10 mm or less) in a case where a constant tension (0.6 N) was applied.
Evaluation C ... deviation of the electrode position was out of an allowable range (more than 10 mm) in a case where a constant tension (0.6 N) was applied.

Materials of each sample of Examples 1 to 7 are as follows. In addition, Table 1 shows each physical property (Poisson's ratio, Young's modulus, maximum thickness, Young's modulus × thickness).

| | |
|---|---|
| Example 1 | polyethylene terephthalate (PET) resin |
| Example 2 | polyimide (PI) resin |
| Example 3 | aluminum foil |
| Example 4 | copper foil |
| Example 5 | polystyrene (PS) resin |
| Example 6 | polyethylene (PE) resin |
| Example 7 | glass film |

In Examples 1 to 6, the deviation of the electrode position in a case where a constant tension was applied was small, and thus the brain wave acquisition could be stably performed at the target position.

In Example 7, the Young's modulus of the support member was smaller than that of Examples 1 to 6, and the thickness was increased as compared with other samples in order to suppress deformation, but the deviation of the electrode in a case where a constant tension was applied was slightly large.

**[Table 1]**

| | Material | Poisson's ratio | Young's modulus [GPa] | Maximum thickness [mm] | Young's modulus x thickness [GPa x mm] | Evaluation |
|---|---|---|---|---|---|---|
| Example 1 | PET | 0.25 to 0.4 | 5 | 0.5 | 2.5 | A |
| Example 2 | PI | 0.29 to 0.30 | 3.4 | 0.225 | 0.765 | A |
| Example 3 | Aluminum foil | 0.345 | 70.3 | 0.05 | 3.515 | A |
| Example 4 | Copper foil | 0.343 | 129.8 | 0.07 | 9.086 | A |
| Example 5 | PS | 0.34 to 0.38 | 2.7 to 4.2 | 0.2 | 1.08 to 2.1 | A |
| Example 6 | Glass film | 0.2 | 73 | 0.03 | 2.19 | A |
| Example 7 | PE | 0.26 | 0.4 to 1.3 | 1 | 0.4 to 1.3 | B |

### <<Verification of Modeling based on Capstan Equation>>

The brain wave measurement device described in the embodiment was mounted on a head model, the electrode pressing force F was measured using a pressure sensor disposed on the surface of the head model, and the measured value was compared with a calculated value obtained by calculation from the model based on the capstan equation.

Specific specifications of the brain wave measurement device 10 are as follows.

Brain wave measurement positions: five locations of T3, C3, Cz, C4, and T4
Electrode unit: base portion diameter of 10 mm
Thickness of base portion of 5 mm
Height of protrusion portion of 5 mm
Number of protrusion portions of 7
Distance between electrodes of 70 mm
Support member (film base material) :
   PI resin
   Poisson's ratio of 0.3
   Young's modulus of 3.4 GPa
   Maximum thickness of 0.225 mm

Instead of fixing the ear attachment portion to the ear, weights of 200 g each were attached to both ends to simulate the mounting state of the brain wave measurement device.

Fig. 10 shows a graph of theoretical values (calculated values) and measured values of the electrode pressing force F at five locations of T3, C3, Cz, C4, and T4. As can be seen from the drawing, it can be confirmed that the theoretical values and the measured values substantially match, and thus the modeling is appropriate.

This application claims priority based on Japanese Patent Application No. 2023-213993 filed on December 19, 2023, the entire disclosure of which is incorporated herein by reference.

### REFERENCE SIGNS LIST

10 brain wave measurement device
20 support member (film base material, ribbon member)
22 circuit pattern
23 protective layer
24 shield layer
25 mounting portion
25a female snap button
30 electrode unit
31 base portion
32 protrusion portion (protruding portion)
33 conductive contact portion (electrode portion)
34 signal line portion
35 male snap button
40 ear attachment portion
50 adjustment part
60 tension display portion
61 spring
62 wire
63 first hook
64 second hook
65 indicator (proper tension indicating portion)
66 housing
67 scale (proper tension indicating portion)
68 indicator section (proper tension indicating portion)
70 attachment part
135 male screw connection terminal
160 electrode attachment part
161 attachment part main body
162 female screw connection terminal

## Claims

1. A brain wave measurement device comprising:
an electrode unit configured to contact a measurement site on a head of a subject and acquire a brain wave signal;
a support member configured to support the electrode unit; and
an assisting member configured to assist placement of the support member on the head,
wherein the support member has a non-stretchable film member and a mounting portion to which the electrode unit is electrically connected and fixed,
the film member has a non-stretchable base material and a circuit pattern provided on the base material, and
the circuit pattern is connected to the mounting portion.

2. The brain wave measurement device according to Claim 1, further comprising:
an electromagnetic wave shielding member configured to cover the circuit pattern.

3. The brain wave measurement device according to Claim 1 or 2,
wherein the film member has an elongated shape, and
a plurality of electrode units are arranged at predetermined intervals in a longitudinal direction of the film member.

4. The brain wave measurement device according to any one of Claims 1 to 3,
wherein the electrode unit has a base portion, a plurality of protruding portions provided on the base portion, and an electrode portion provided on the protruding portion and in contact with the head.

5. The brain wave measurement device according to Claim 4,
wherein the protruding portions are elastic members.

6. The brain wave measurement device according to any one of Claims 1 to 5,
wherein the assisting member is provided at an end portion of the support member, is configured to be attached to an ear or a jaw, and assists placement of the support member along a shape of the head.

7. The brain wave measurement device according to any one of Claims 1 to 6,
wherein the assisting member includes a member having spring properties.

8. The brain wave measurement device according to any one of Claims 1 to 7,
wherein the assisting member includes a member for adjusting a length.

9. The brain wave measurement device according to any one of Claims 1 to 8,
wherein the support member is bent at a vertex and linearly connects the vertices, each attachment position of the electrode unit serving as the vertex.

10. A brain wave measurement method comprising:
mounting the brain wave measurement device according to any one of Claims 1 to 9 on a head of a subject and measuring brain waves.
